# EUROPEAN PATENT SPECIFICATION

(11) **EP 4 324 914 B1**
(45) Date of publication and mention of the grant of the patent: **18.03.2026**
(21) Application number: 21945198.6
(22) Date of filing: 11.06.2021
(51) Int. Cl.: C12N 5/0775, A61K 35/12, C12N 5/00

(54) **EXOSOME PRODUCTION PROMOTING AGENT AND EXOSOME PRODUCTION PROMOTING METHOD**
MITTEL ZUR FÖRDERUNG DER EXOSOMPRODUKTION UND VERFAHREN ZUR FÖRDERUNG DER EXOSOMPRODUKTION
AGENT FAVORISANT LA PRODUCTION D'EXOSOME ET PROCÉDÉ FAVORISANT LA PRODUCTION D'EXOSOME

(43) Date of publication of application: 21.02.2024
(73) Proprietor: Fullstem Co., Ltd., Naha-shi, Okinawa 900-0005 (JP)
(72) Inventor: CHIBA, Shunmei, Naha-shi, Okinawa 900-0005 (JP)
(74) Representative: Germain Maureau
(86) International application number: PCT/JP2021/022314
(87) International publication number: WO 2022/259526

(56) References cited:
- WO-A1-2018/182356
- WO-A1-2018/182356
- WO-A1-2019/088656
- WO-A1-2019/168000
- KR-A- 20180 111 674
- KR-A- 20180 111 674
- US-A1- 2019 133 922
- US-A1- 2019 133 922
- DIEM HUONG HOANG ET AL: "Differential Wound Healing Capacity of Mesenchymal Stem Cell-Derived Exosomes Originated From Bone Marrow, Adipose Tissue and Umbilical Cord Under Serum- and Xeno-Free Condition", FRONTIERS IN MOLECULAR BIOSCIENCES, vol. 7, 24 June 2020 (2020-06-24), XP055751611, DOI: 10.3389/fmolb.2020.00119
- ANONYMOUS WEIZHI ET AL: "Epidermal growth factor (EGF) and interleukin (IL)-1[beta] synergistically promote ERK1/2-mediated invasive breast ductal cancer cell migration and invasion | Molecular Cancer | Full Text", ADVANCED MATERIALS, vol. 30, no. 22, 21 October 2012 (2012-10-21), DE, pages e1800106 - n/a, XP093227922, ISSN: 0935-9648, Retrieved from the Internet <URL:https://molecular-cancer.biomedcentral.com/articles/10.1186/1476-4598-11-79> DOI: 10.1002/adma.201800106
- TOMOHIRO KATO;SHIGERU MIYAKI;HIROYUKI ISHITOBI;YOSHIHIRO NAKAMURA;TOMOYUKI NAKASA;MARTIN K LOTZ;MITSUO OCHI: "Exosomes from IL-1beta stimulated synovial fibroblasts induce osteoarthritic changes in articular chondrocytes", ARTHRITIS RESEARCH AND THERAPY, BIOMED CENTRAL, LONDON, GB, vol. 16, no. 4, 4 August 2014 (2014-08-04), GB , pages R163, XP021193643, ISSN: 1478-6354, DOI: 10.1186/ar4679
- ANONYMOUS: "The key to mass culture of stem cells was "non-woven fabric"! Regenerative Medicine Venture "Full Stem" Challenged by Brain Surgeons (ICC FUKUOKA 2020) [Transcript]", ICC SUMMIT FUKUOKA 2023, 21 June 2020 (2020-06-21), XP093017627, Retrieved from the Internet <URL:https://industry-co-creation.com/catapult/56800> [retrieved on 20230125]

## Description

### TECHNICAL FIELD

The present invention relates to an exosome production promoter that promotes production of exosomes from a mesenchymal stem cell, and a method for promoting the production of exosomes.

### BACKGROUND ART

Mesenchymal stem cells (MSCs) are multipotent stem cells present in mesenchymal tissues, i.e., mesoderm-derived connective tissues. It has also been revealed that undifferentiated mesenchymal stem cells secrete cytokines and growth factors having an antiinflammatory effect, a cell-proliferation-promoting effect, an angiogenesis-promoting effect, and the like, and support tissue repair via paracrine (NON-PATENT DOCUMENT 1). A molecular group secreted by the undifferentiated mesenchymal stem cells has therapeutic effects on various diseases not limited to a specific disease. Tissue regeneration becomes possible by using naive mesenchymal stem cells without inducing differentiation of the mesenchymal stem cells into cells of a tissue to be repaired and without adding any artificial operation such as genetic recombination to the cells.

Exosomes can mediate intercellular communication by transferring proteins, lipids and RNAs between cells (NON-PATENT DOCUMENTS 2 and 3). It has been revealed that molecules such as those of proteins, microRNAs, and mRNAs contained in exosomes have functions similar to those of cells from which the exosomes are derived. Therefore, exosomes secreted by mesenchymal stem cells, which have attracted attention as a source of cell therapy for a wide range of diseases, are expected to have therapeutic effects similar to those of MSCs (NON-PATENT DOCUMENT 4).

PATENT DOCUMENT 1 describes exosomes isolated from a cultured neural stem cell line (NSCL). The exosomes can promote migration of fibroblasts, branching of human umbilical vein endothelial cells, and extension of neurites.

Exosomes contain relatively less animal serum than stem cells, and can eliminate the risk of symptoms due to animal serum infection. Therefore, cell therapy using exosomes can overcome the limitations of existing stem cell therapies.

PATENT DOCUMENT 2 discloses an exosome production promoter containing a sphingoid base as an active ingredient. However, the exosome production promoter described in PATENT DOCUMENT 2 is limited to those capable of promoting production of exosomes in nerve cells in order to cure Alzheimer's disease.

### CITATION LIST

### PATENT DOCUMENTS

PATENT DOCUMENT 1: WO 2013/150303 A
PATENT DOCUMENT 2: Japanese Unexamined Patent Publication No. 2019-156786

### NON-PATENT DOCUMENTS

NON-PATENT DOCUMENT 1: Chamberlain G, Fox J, Ashton B, Middleton J, Concise review: mesenchymal stem cells: their phenotype, differentiation capacity, immunological features, and potential for homing. Stem Cells. 25 (2007) 2739-2749
NON-PATENT DOCUMENT 2: Simons and Raposo, Curropin Cell Biology 2009; 21:575-581
NON-PATENT DOCUMENT 3: Skog et al., Nat Cell Biology 2008; 10:1470-1476; Valadi et al., Nat Cell Biology 2007; 9:654-659
NON-PATENT DOCUMENT 4: Katsuda T, Kosaka N, Takeshita F, Ochiya T. The therapeutic potential of mesenchymal stem cell-derived extracellular vesicles. Proteomics. 13 (2013) 1637-1653.

### SUMMARY OF THE INVENTION

### TECHNICAL PROBLEM

The present invention has been made in view of such problems, and an object of the present invention is to provide an exosome production promoter that promotes production of exosomes from adipose-derived mesenchymal stem cells, and an exosome production promoting method.

### SOLUTION TO THE PROBLEM

An exosome production promoter according to the present invention is an exosome production promoter that promotes production of exosomes from adipose-derived mesenchymal stem cells cultured in a serum-free medium, and contains EGF.

An exosome production promoting method according to the present invention is an exosome production promoting method for promoting production of exosomes from adipose-derived mesenchymal stem cells, the method including culturing adipose-derived mesenchymal stem cells in a serum-free medium containing EGF.

### ADVANTAGES OF THE INVENTION

According to the present invention, it is possible to promote the production of exosomes from adipose-derived mesenchymal stem cells.

### BRIEF DESCRIPTION OF THE DRAWING

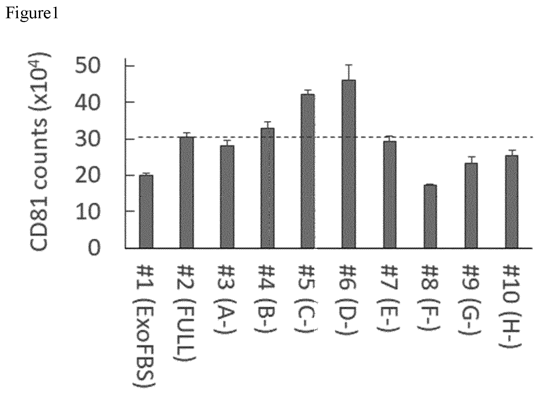

FIG. 1 is a diagram showing amounts of exosomes produced from adipose-derived mesenchymal stem cells when various factors are added.

### DESCRIPTION OF EMBODIMENT

Embodiments of the present invention will be described in detail below while referring to the accompanying drawings. The embodiments are only intended to facilitate the understanding of the principles of the present invention, and the scope of the present invention is not limited to the following embodiments.

The exosome production promoter according to the present invention promotes the production of exosomes from adipose-derived mesenchymal stem cells cultured in a serum-free medium.

The adipose-derived mesenchymal stem cells are preferably human adipose-derived mesenchymal stem cells. Adipose-derived mesenchymal stem cells have advantageous features such as more rapid cell proliferation, increased secretion of regeneration-promoting factors, and higher immunosuppressive ability, as compared with bone marrow-derived mesenchymal stem cells. Another advantageous feature of adipose-derived mesenchymal stem cells is that it is easier to obtain a sufficient amount of adipose-derived mesenchymal stem cells from adipose tissue of the abdomen or buttocks in a safe manner, as compared with bone marrow-derived mesenchymal stem cells for which bone marrow needs to be collected. In addition, the use of mesenchymal stem cells derived from autologous adipose tissue of a patient for treatment of the patient is superior in terms of having no ethical problem, no immune rejection, few problems such as infection, and a therapeutic effect when administered intravenously.

Adipose-derived mesenchymal stem cells are positive for, for example, CD73, CD90, CD105, and CD44, and negative for CD11b, CD19, CD34, CD45, and HLA-DR. Adipose tissue-derived mesenchymal stem cells have an ability to differentiate into, for example, osteoblasts, adipocytes, and chondrocytes.

Adipose tissue-derived mesenchymal stem cells are obtained in the primary culture of mesenchymal stem cells which are separated from adipose tissue, in a serum-containing medium, and then by proliferating and culturing the mesenchymal stem cells passaged from the primary culture in a serum-free medium. The adipose tissue is obtained by surgical resection, for example, from a mammal such as a human. At the time of surgical resection, local anesthesia may be performed. Alternatively, the adipose tissue may be obtained by aspiration with a catheter inserted into subcutaneous adipose tissue of the abdomen, thigh, or buttocks. The amount of adipose tissue obtained is, for example, but not limited to, 1 g to 100 g, 2 g to 50 g, 2 g to 40 g, or 2 g to 20 g.

The obtained adipose tissue is washed with, for example, physiological saline, and immersed in a solution containing collagenase diluted at a concentration of 0.1% in a Hank's balanced salt solution, at 37°C for 70 minutes, for dispersion. Adipose tissue-derived cells dispersed from the adipose tissue are centrifuged at 1800 rpm for 10 minutes. Further, a stromal vascular fraction obtained by centrifugation is diluted with the solution and filtered through a 100 µm nylon mesh. The filtered cells are washed with MEMα.

The serum-free medium is a serum-free medium for mesenchymal stem cells, and examples thereof include DMEM, MEMα, DMEM/F12, MEM, and the like.

The exosome production promoter according to the present invention contains EGF. As shown in Examples described later, the amount of exosomes produced from adipose-derived mesenchymal stem cells in a case of containing a plurality of factors is larger than the amount of exosomes produced from adipose-derived mesenchymal stem cells in a case of containing factors excluding only EGF from the plurality of factors. The inventor of the present invention has found, as a new finding, that EGF is a factor that promotes the production of exosomes, and has completed the present invention based on this fact.

EGF is a 6045 Da protein consisting of 53 amino acid residues and three intramolecular disulfide bonds. EGF binds as a ligand to epidermal growth factor receptor (EGFR) present on the cell surface and plays an important role in regulation of growth and proliferation of cells. The content of EGF in the medium is not particularly limited, and is preferably 0.05 ng/ml to 300 ng/ml in terms of final concentration, and more preferably 1 ng/ml to 100 ng/ml in terms of final concentration.

In addition, the exosome production promoter preferably contains IL-1β as well since IL-1β, too, promotes the amount of exosomes produced from adipose-derived mesenchymal stem cells. The content of IL-1β in the medium is not particularly limited, and is preferably 0.001 ng/ml to 10 ng/ml in terms of final concentration, and more preferably 0.01 ng/ml to 1 ng/ml in terms of final concentration.

Moreover, the exosome production promoter preferably contains trehalose as well since trehalose, too, promotes the amount of exosomes produced from adipose-derived mesenchymal stem cells. Trehalose is a disaccharide formed by a 1,1-glycosidic bond of two α-glucose. The content of trehalose in the medium is not particularly limited, and is preferably 1 mg/ml to 50 mg/ml in terms of final concentration, and more preferably 10 mg/ml to 30 mg/ml in terms of final concentration.

It is desirable that the exosome production promoter according to the present invention does not contain SCGF. As shown in Examples described later, the amount of exosomes produced from adipose-derived mesenchymal stem cells in a case of containing a plurality of factors is smaller than the amount of exosomes produced from adipose-derived mesenchymal stem cells in a case of containing factors excluding only SCGF from the plurality of factors. The inventor of the present invention has found, as a new finding, that SCGF is a factor that inhibits the production of exosomes. Therefore, it is preferable that the exosome production promoter according to the present invention does not contain SCGF. SCGF is a 29-kDa protein having no N-type sugar chain and belongs to a C-type lectin family.

It is also preferable that the exosome production promoter according to the present invention does not contain TNFα since TNFα, too, inhibits exosome production from adipose-derived mesenchymal stem cells.

In the present invention, it is preferable that the adipose-derived mesenchymal stem cells are cultured using a nonwoven fabric as a scaffold. The production of exosomes is promoted when the adipose-derived mesenchymal stem cells are cultured using a nonwoven fabric as a scaffold.

The mass of the nonwoven fabric per unit area may be 1 g/m² to 500 g/m², and preferably 50 g/m² to 200 g/m², for example. The nonwoven fabric may be a hydrophilized nonwoven fabric. The nonwoven fabric can be hydrophilized by a fluorine gas treatment, an atmospheric pressure plasma treatment, a vacuum plasma treatment, a corona treatment, a graft polymerization treatment of a hydrophilic monomer, a sulfonation treatment, or a surfactant application treatment, and preferably by the fluorine gas treatment or the atmospheric pressure plasma treatment.

It is preferable that fibers constituting the nonwoven fabric are a polyolefin-based polymer. Examples of the polyolefin-based polymer include ethylene, propylene, 1-butene, 1-pentene, 3-methyl-1-butene, 1-hexene, 1-octene, 1-dodecene, 1-octadecene, etc.

The fibers constituting the nonwoven fabric are desirably fibers having a small fiber diameter. The average fiber diameter is preferably 200 µm or less, more preferably 010 µm to 100 µm, and particularly preferably 15 µm to 50 µm. The fibers may be a mixture of fibers having a relatively large fiber diameter and fibers having a relatively small fiber diameter.

Preferably, the nonwoven fabric woven fabric is porous. Porosity is important to supply sufficient amounts of oxygen and nutrients to cells for tissue regeneration and to quickly remove carbon dioxide and waste products. In addition, having porosity increases a specific surface area and enhances cell adhesion. The average pore diameter in such a case is, for example, 5 µm to 200 µm, 20 µm to 100 µm, 25 µm to 100 µm, 30 µm to 100 µm, 35 µm to 100 µm, 40 µm to 100 µm, 50 µm to 100 µm, or 60 µm to 100 µm, and preferably 50 µm to 300 µm.

The form of the nonwoven fabric is not particularly limited, but preferably a nonwoven fabric sheet. It is preferable that the nonwoven fabric sheet has a plurality of through holes penetrating in the thickness direction. The total film thickness of the nonwoven fabric sheet may be, for example, 5 µm or more, 10 µm or more, 20 µm or more, or 25 µm or more, and may be 500 µm or less, 300 µm or less, 100 µm or less, 75 µm or less, or 50 µm or less. The total film thickness is preferably 30 µm to 2000 µm, and more preferably 500 µm to 1000 µm.

The exosome produced by using the exosome production promoter according to the present invention can be used as an exosome-containing formulation. The form of the exosome-containing formulation is not particularly limited, and may be, for example, liquids, such as an injection, a suspension, a solution, and a spray, a sheet-like formulation, or a gel-like formulation.

The exosome-containing formulation can be used without limitations, and can be used, for example, for direct administration into a subject, or as a source for reconstruction of tissues and organs in vitro.

The exosome-containing formulation can be used to treat diseases and disorders. Examples of the diseases and disorders that can be a target include immunologic disease, ischemic disease (such as lower limb ischemia, ischemic heart disease (such as myocardial infarction), coronary heart disease, cerebrovascular ischemia, renal ischemia, and pulmonary ischemia), neurological disease, Crohn's disease, graft-versus-host disease (GVHD), inflammatory bowel disease including ulcerative colitis, collagen diseases including systemic lupus erythematosus, hepatic cirrhosis, cerebral infarction, intracerebral hematoma, cerebral vasospasm, radiation enteritis, atopic dermatitis, multiple sclerosis, rheumatoid arthritis, psoriasis, lupus erythematosus, diabetes, mycosis fungoides (Alibert-Bazin syndrome), scleroderma, diseases caused by degeneration and/or inflammation of connective tissues such as cartilage, eye disease, angiogenesis related disease, congestive heart failure, cardiomyopathy, wound, epithelial damage, fibrosis, pulmonary disease, and cancer.

The exosome-containing formulation may also be used for treatment, care, or improvement for cosmetic purposes. Cosmetic purposes not only are the pure cosmetic purposes for the healthy state, but also include cosmetic treatments for post-surgical or post-traumatic deformities and congenital deformities. For example, the exosome-containing formulation can be used for breast tissue augmentation (breast augmentation, breast reconstruction), tissue augmentation of cheek or sunken upper and lower eyelids, and tissue augmentation for facial hemiatrophy and for face or funnel chest.

The exosome-containing formulation may contain a pharmaceutically acceptable carrier or additive besides exosomes. Examples of such a carrier or additive include, but are not limited to, a tonicity agent, a thickener, saccharides, sugar alcohols, an antiseptic (preservative), a fungicide or antibiotic, a pH adjusting agent, a stabilizing agent, a chelating agent, an oleaginous base, a gel base, a surfactant, a suspending agent, a binder, a filler, a lubricant, a disintegrant, a foaming agent, a glidant, a dispersant, an emulsifier, a buffer, a solubilizer, an antioxidant, a sweetening agent, an acidulant, a colorant, a taste making agent, a flavor, and a cooling agent.

According to the present invention, it is possible to supply a large amount of exosomes stably from adipose-derived mesenchymal stem cells. Application to various treatments and cosmetics is also possible by making molecules having therapeutic effects, such as mRNAs, miRNAs, and proteins, overexpressed in mesenchymal stem cells using a genetic recombination technology and producing exosomes carrying these molecules. It is also possible to deliver exosomes to a target tissue more specifically, by modifying the surface of the exosomes by, for example, causing a receptor for a protein that is expressed specifically in the target tissue to be expressed on the surface of the exosomes using a genetic recombination technology.

### [Examples]

4 × 10⁴ adipose-derived mesenchymal stem cells (ADSC) were seeded in each well. A nonwoven fabric (3D, BioNOCII, CESCO BIOENGINEERING CO., LTD. in non-adhesive 24 well plate (PrimeSurface (registered trademark) Sumitomo Bakelite Co., Ltd.) was used for cell culture. A basal medium was 1.5 ml of 10% FBS/DMEM F12 (Sigma-Aldrich). The number of days of culture was 6. Starting from Day 2, 1.5 ml of medium was exchanged daily, and the amount of sugar consumed in the used culture medium was measured.

Next, in the cell culture for 48 hours on Day 7 and Day 8, DMEM F12 was used as a medium so as to collect exosomes. In this 48-hour culture, 10% ExoFBS (Funakoshi Co., Ltd.) was added in Test #1. ExoFBS is a product from which bovine-derived exosomes are removed; therefore, influence on an exosome research is reduced. In Test #2 of the 48-hour culture, all eight factors A to H were added at the concentrations shown in Table 1 below.

**[Table 1]**

| | Factor | Concentration |
|---|---|---|
| A | IGF-1 | 10 ng/ml |
| B | bFGF | 10 ng/ml |
| C | TNFα | 1 ng/ml |
| D | SCGF | 1 ng/ml |
| E | PDGF | 1 ng/ml |
| F | EGF | 10 ng/ml |
| G | IL-1β | 1 ng/ml |
| H | Trehalose | 10 mg/ml |

In Test #3 of the 48-hour culture, only factor A was excluded from the eight factors shown in Table 1, and the other factors were added. In Test #4 of the 48-hour culture, only factor B was excluded from the eight factors shown in Table 1, and the other factors were added. In Test #5 of the 48-hour culture, only factor C was excluded from the eight factors shown in Table 1, and the other factors were added. In Test #6 of the 48-hour culture, only factor D was excluded from the eight factors shown in Table 1, and the other factors were added. In Test #7 of the 48-hour culture, only factor E was excluded from the eight factors shown in Table 1, and the other factors were added. In Test #8 of the 48-hour culture, only factor F was excluded from the eight factors shown in Table 1, and the other factors were added. In Test #9 of the 48-hour culture, only factor G was excluded from the eight factors shown in Table 1, and the other factors were added. In Test #10 of the 48-hour culture, only factor H was excluded from the eight factors shown in Table 1, and the other factors were added.

The amount of exosomes produced from ADSC was measured with Exo Counter (Kenwood), using 50 µl each from 1.5 ml of the collected culture supernatant. Exo Counter detects exosomes by sandwich immunoassay using disc and nanobeads antibodies in a surface antigen-specific manner. CD81 on the exosome surface was detected to quantify the exosome amount.

The results are shown in FIG. 1. As shown in FIG. 1, the amount of exosomes produced was reduced in the case where EGF was excluded, and it was found that EGF was a factor that promotes the production of exosomes. The amount of exosomes produced was reduced also in the case where IL-1β was excluded, and it was found that IL-1β was another factor that promoted the production of exosomes. The amount of exosomes produced was reduced also in the case where trehalose was excluded, and it was found that trehalose was another factor that promoted the production of exosomes.

Conversely, the amount of exosomes produced increased in the case where SCGF was excluded, and it was found that SCGF was a factor that inhibits the production of exosomes. The amount of exosomes produced increased also in the case where TNFα was excluded, and it was found that TNFα was another factor that inhibited the production of exosomes.

### INDUSTRIAL APPLICABILITY

The exosome production promoter can be used for the production of exosomes.

## Claims

1. A serum-free medium that promotes production of exosomes from adipose-derived mesenchymal stem cells, the serum-free medium containing EGF, IL-1β and trehalose.

2. The serum-free medium of claim 1, wherein the serum-free medium does not contain SCGF.

3. The serum-free medium of any one of claims 1 to 2, wherein the serum-free medium does not contain TNFα.

4. An exosome production promoting method for promoting production of exosomes from adipose-derived mesenchymal stem cells, the method comprising
culturing the adipose-derived mesenchymal stem cells in a serum-free medium containing EGF, IL-1β and trehalose.

5. The exosome production promoting method of claim 4 wherein the serum-free medium does not contain SCGF.

6. The exosome production promoting method of any one of claims 4 to 5, wherein the serum-free medium does not contain TNFα.

7. The exosome production promoting method of any one of claims 4 to 6, wherein the adipose-derived mesenchymal stem cells are cultured using a nonwoven fabric as a scaffold.

## Patentansprüche

1. Serumfreies Medium, das die Produktion von Exosomen von aus Fettgewebe gewonnenen mesenchymalen Stammzellen fördert, wobei das serumfreie Medium EGF, IL-1β und Trehalose enthält.

2. Serumfreies Medium nach Anspruch 1, wobei das serumfreie Medium kein SCGF enthält.

3. Serumfreies Medium nach einem der Ansprüche 1 bis 2, wobei das serumfreie Medium kein TNFα enthält.

4. Exosomenproduktionsförderndes Verfahren zur Förderung der Produktion von Exosomen aus Fettgewebe gewonnenen mesenchymalen Stammzellen, wobei das Verfahren
das Kultivieren der aus Fettgewebe gewonnenen mesenchymalen Stammzellen in einem serumfreien Medium umfasst, das EGF, IL-1β und Trehalose enthält.

5. Exosomenproduktionsförderndes Verfahren nach Anspruch 4, wobei das serumfreie Medium kein SCGF enthält.

6. Exosomenproduktionsförderndes Verfahren nach einem der Ansprüche 4 bis 5, wobei das serumfreie Medium kein TNFα enthält.

7. Exosomenproduktionsförderndes Verfahren nach einem der Ansprüche 4 bis 6, wobei die aus Fettgewebe gewonnenen mesenchymalen Stammzellen unter Verwendung eines Vliesstoffs als Gerüst kultiviert werden.

## Revendications

1. Milieu sans sérum qui favorise la production d'exosomes à partir de cellules souches mésenchymateuses dérivées du tissu adipeux, le milieu sans sérum contenant EGF, IL-1β et du tréhalose.

2. Milieu sans sérum selon la revendication 1, dans lequel le milieu sans sérum ne contient pas de SCGF.

3. Milieu sans sérum selon l'une quelconque des revendications 1 et 2, dans lequel le milieu sans sérum ne contient pas de TNFα.

4. Procédé favorisant la production d'exosomes pour favoriser la production d'exosomes à partir de cellules souches mésenchymateuses dérivées du tissu adipeux, le procédé comprenant
la mise en culture des cellules souches mésenchymateuses dérivées du tissu adipeux dans un milieu sans sérum contenant EGF, IL-1β et du tréhalose.

5. Procédé favorisant la production d'exosomes selon la revendication 4, dans lequel le milieu sans sérum ne contient pas de SCGF.

6. Procédé favorisant la production d'exosomes selon l'une quelconque des revendications 4 et 5, dans lequel le milieu sans sérum ne contient pas de TNFα.

7. Procédé favorisant la production d'exosomes selon l'une quelconque des revendications 4 à 6, dans lequel les cellules souches mésenchymateuses dérivées du tissu adipeux sont mises en culture en utilisant un tissu non tissé comme échafaudage.
